# EUROPEAN PATENT APPLICATION

(11) **EP 0 569 629 A1**
(43) Date of publication of application: **18.11.1993**
(21) Application number: 92304209.7
(22) Date of filing: 11.05.1992
(51) Int. Cl.: C05C 9/00, C07C 273/16

(54) **Method for producing acidic urea solutions**

(71) Applicant: Union Oil Company of California, dba UNOCAL, Los Angeles, CA 90017 (US)
(72) Inventor: Brown, Roger Allen, Chino Hills, California (US); Young, Donald Clifford, Fullerton, California (US)
(74) Representative: Enskat, Michael Antony Frank

(57) **Abstract**

Acidic urea solutions having low equilibrium vapor pressures are produced by stripping an aqueous urea solution having a pH of about 5.5 or less and a total carbonate content which, if present as carbon dioxide, would produce an equilibrium vapor pressure over said solution of more than 0.5 psig. at 20° C., under conditions sufficient to reduce the total carbonate content to a level which, if present as carbon dioxide, would produce an equilibrium vapor pressure over the solution of 0.5 psig. or less at 20° C. Methods for storing such compositions are also provided.

## Description

The invention relates to acidic urea solutions and, in particular, to methods for producing acidic urea solutions having low equilibrium vapor pressures.

Urea is widely used in several industries as a chemical precursor, fertilizer, feed supplement for ruminant mammals and is a component of various mixtures such as dermal salves and creams. A large volume of urea is employed in the agricultural industry as a fertilizer and protein nitrogen source for ruminant mammals, and much of this is used in the form of aqueous solutions, including dispersions containing insoluble matter. In many instances, it is preferable to acidify urea solutions to increase the solubility of otherwise insoluble materials such as zinc, iron and manganese nutrients as well as various phosphates employed in the agricultural and chemical industries. Acidified urea solutions are also employed to counteract alkalinity of water supplies with which the solution may be mixed and/or to counteract alkalinity of alkaline soils prevalent in the western United States.

A plant nutrient combination ideal for many applications is a solution containing sufficient available phosphorus and nitrogen to supplement or completely supply plant needs. Phosphoric acid is one of the most readily available and least expensive sources of water-soluble available phosphate. However, ammonium salts have only limited solubility in phosphoric acid solutions, and nitrates are converted, in the presence of phosphoric acid, to nitric acid which is unacceptable to many manufacturers and users. Urea, on the other hand, is both stable and highly soluble in phosphoric acid. Thus, aqueous solutions of urea and phosphoric acid are ideal for many plant nutrient applications, and concentrated solutions of these two components are particularly desirable for economic reasons, e.g. cost of shipment, handling, etc. Both dilute and concentrated solutions of urea and sulfuric acid have also found wide acceptance in the agricultural industry as fertilizers, soil adjuvants and herbicides. Such fertilizers and their methods of manufacture are disclosed by Donald C. Young in U.S. Patents 4,445,925, METHODS OF PRODUCING CONCENTRATED UREA-SULFURIC ACID REACTION PRODUCTS, issued May 1, 1984 and 4,447,253, TOPICAL FERTILIZATION METHODS AND COMPOSTIIONS FOR USE THEREIN, issued May 8, 1984, the disclosures of which are incorporated herein in their entireties.

While the benefits of using acidified urea solutions in many chemical and agricultural applications have been recognized for years, most often it has proven impossible to obtain such solutions which do not generate such elevated vapor pressures that they cannot be stored in closed containers. And, while this problem has not prevented the use of acidic urea solutions in large volume, industrial applications, it has proven to be a significant problem in smaller containers employed in industry and, in particular, in the home and garden market where inversion of vented containers is highly probable. In view of this problem, a significant market in vented containers, designed specifically for acidic urea solutions, has grown up due to the demand of the home and garden market. While such containers do prevent the problems of container expansion and explosion which occurs with unvented containers, their use does not prevent spillage, and it raises yet another problem when surfactant-containing solutions are employed. Surfactants are often used in urea fertilizers to facilitate distribution, and they are also employed in various industrial applications such as the promotion of acid-catalyzed reactions with urea-sulfuric acid compositions containing the monourea adduct of sulfuric acid as disclosed by Donald C. Young in U.S. Patent 4,722,986, ACID CATALYSTS AND METHODS OF USE, issued February 2, 1988, the disclosure of which is incorporated herein in its entirety. The use of surfactants in such acidic urea solutions in vented containers poses a significant problem of leakage and foaming due to foam formation as the solution off-gases during storage.

For years, it has been believed that the high vapor pressure characteristic of acidic urea solutions was due to either chemical or enzymatic hydrolysis during storage, either of which would be persistent throughout the solution's storage life, and this belief was due primarily to several observations. First, if unvented containers of acidic urea solutions stored for a period sufficient to build significant pressure, e.g. one day or more, are opened to release the accumulated gas and reclosed, they will build pressure upon further storage. Secondly, some investigators reported limited, infrequent success in reducing pressure buildup by adding to the acidic urea solutions chemicals which denature urease enzyme. In addition, it was noted that the pressure buildup associated with such solutions was due primarily, if not exclusively, to the accumulation of carbon dioxide, a urea hydrolysis product. Nevertheless, the problem of persistent pressure buildup in unvented containers of acidified urea solutions still remains, and its remedy would benefit several industries.

It has now been found that the persistent vapor pressure buildup over acidic urea solutions is due to the presence of carbonates, apparently introduced with the urea, in combination with unusual kinetic and equilibrium behavior of carbonate in such solutions. It has also been found that the vapor pressure buildup of stored acidic urea solutions can be alleviated or eliminated by stripping the solutions under conditions sufficiently severe to reduce the total carbonate content to a level which, if all present as carbon dioxide, does not result in an unacceptable equilibrium vapor pressure even after prolonged storage. This finding, i.e. that the problem of vapor pressure buildup is associated with the presence of carbonate, was not expected since carbonates typically decompose immediately to carbon dioxide upon acidification, and the resulting carbon dioxide is immediately evolved from the solution. In fact, rapid carbon dioxide evolution from basic urea solutions during neutralization with acid was observed to be so rapid by Donald C. Young in U.S. Patent 4,345,099, METHOD OF SELECTIVELY REMOVING BIURET FROM UREA AND COMPOSITIONS FOR USE THEREIN, issued August 17, 1982, that Young advised the exercise of caution during acid addition for the reason that, in that instance, carbonate neutralization caused carbon dioxide evolution at a rate so rapid that it could produce foaming and volume expansion. (See, in particular, column 9, lines 7-17.) While rapid CO₂ evolution did occur during acidification of the alkaline urea solutions described in U.S. Patent 4,345,099, it has been found that such rapid conversion and carbon dioxide evolution from solutions containing the minor amounts of carbonate present in most commercial ureas, or a residual minor amount of carbonate remaining after evolution of the principal amount of carbon dioxide generated by acid neutralization, does not occur. In fact, acidified urea solutions can be vented numerous times and will continue to build pressure in a closed container.

Without intending to be constrained to any particular theory, it is presently believed that the relatively severe stripping employed in these methods either favorably shifts an equilibrium otherwise existing in the solution and/or increases the rate of carbonate conversion to carbonic acid and carbon dioxide sufficiently to reduce total carbonate content to a level that does not result in unacceptable equilibrium vapor pressures. For the purposes of this invention, "total carbonate content" is intended to include carbonates, bicarbonates, carbonic acid and carbon dioxide. "Equilibrium vapor pressure" is intended to mean the vapor pressure existing over the solution in a closed container in which 90 percent of the volume is occupied by the solution after storage for 48 hours. Herein, equilibrium vapor pressure is expressed and measured at 20° C. Obviously, the equilibrium vapor pressure of these solutions, as with all vaporizable solutions, increases as temperature is increased.

The resulting solutions have significantly improved storage stability in that they do not develop significant equilibrium vapor pressures even after prolonged storage. Thus, they can be stored in closed containers, thereby avoiding the added cost and the hazards and inconvenience of storage in vented containers such as spillage, foaming (when surfactants are present) and contamination.

Acidic, aqueous urea solutions having acceptable equilibrium vapor pressure during storage in closed containers can be obtained from solutions having a pH of 5.5 or less and a total carbonate content which, if present as carbon dioxide, would produce an equilibrium vapor pressure of more than 0.5 psig. at 20° C. by stripping such solutions under conditions sufficiently severe to reduce the total carbonate content to a level which, if present as carbon dioxide, would produce an equilibrium vapor pressure of 0.5 psig. or less at 20° C. Adequate stripping can be achieved by sufficiently severe gas stripping and/or mechanical means, preferably by a combination of the two. The gases used for this purpose are preferably, relatively insoluble in the solution and include air, nitrogen, and other chemically inert gases. The gas can be introduced into the solution by any suitable means such as sparging into the liquid or passage upwardly through a packed tower through which the solution is passed downwardly. Other procedures known in the art for stripping liquids with gases are also suitable.

Mechanical stripping can be achieved by severe agitation with either mechanical means such as stirrers or impellers, by contact with high surface area media such as packed columns, or by rapid gas injection into the liquid through a gas distribution means. Combined gas and mechanical stripping are particularly preferred, and this can be achieved by the use of packed columns as described above or by agitating the solution by mechanical means combined with sparging a stripping gas into the solution.

The time required for adequate stripping depends, to a large extent, on the severity of mechanical and/or gas stripping employed, and should be sufficient to reduce the total carbonate content to a level which, if present as carbon dioxide, would produce an equilibrium vapor pressure over the solution of 0.5 psig. or less at 20° C. Typically, stripping will be continued for at least about 1 minute, generally at least about 2 minutes and can involve about 2 minutes to several hours, e.g. 2 hours or more depending on the efficiency (severity) of the stripping procedure involved. The exact time required for a given stripping procedure to achieve the necessary reduction in total carbonate content can be readily determined by subjecting separate samples of the solution to stripping by that procedure for various lengths of time, e.g. 1 minute, 10 minutes, 1 hour, etc., storing the sample solutions in closed containers in which the solution occupies 90 percent of the container volume, and measuring the vapor pressure over the solution after a selected period of time, e.g. 48 hours, 1 week, or longer period as desired. The total quantity of solution can then be stripped for the time required to achieve the desired reduction in equilibrium vapor pressure and, if desired, can be stored in either closed or open containers.

The temperature of the solution during stripping is not critical, and adequate stripping can be achieved at ambient temperature, e.g. 20° C. However, as disclosed by Young in U.S. Patent 4,445,925, referred to above, temperatures of 70° C. (158° F.) should not be exceeded when employing relatively concentrated urea-sulfuric acid compositions having urea/H₂SO₄ molar ratios above 2, and temperatures of 80° C. (176° F.) should not be exceeded with compositions having urea/H₂SO₄ molar ratios below 2, since those solutions tend to decompose above those respective temperatures. Furthermore, stripping can be accelerated at elevated temperature, i.e. up to 70° C. Thus, stripping temperatures are usually within the range of about 20 to about 60° C.

The solutions can contain any mineral or organic acid capable of obtaining a pH of about 5.5 or less in the urea solutions. Illustrative acids include sulfuric, nitric, phosphoric, hydrochloric, citric, acetic, etc., and combinations of one or more mineral or organic acids can be employed. The mineral acids are presently preferred, with sulfuric and phosphoric acids being particularly preferred. Acid concentration is sufficient to obtain a pH of about 5.5 or less, usually about 5 or less, and most often about 2 or less. (Equilibrium vapor pressure buildup with solutions having pH levels above 5.5 is less evident than with solutions having pH levels of 5.5 and less.) The acid concentration required to achieve these pH levels, of course, depends upon the relative acid strength of the acid and usually will be at least about 0.5, typically at least about 1, preferably at least about 5, and most preferably at least about 10 weight percent. Thus, acid concentrations within the range of about 0.5 weight percent to saturation can be employed, and these usually correspond to about 0.5 to 60 weight percent depending on the acid selected, urea concentration, and the presence and concentration of other components.

Urea concentration can also vary considerably and is usually within the range of about 5 weight percent up to the solubility limit of urea in the solution. However, since the problem of gradual carbon dioxide evolution and consequent vapor pressure buildup in closed containers is more severe in the more concentrated solutions, these methods are particularly useful for treating solutions containing at least about 10 weight percent urea, and are even more beneficial at higher urea concentrations, e.g. about 20 weight percent and above.

The acidic urea solutions to be treated by the described stripping procedures will generally have an equilibrium vapor pressure above 0.5 psig. since vapor pressures of 0.5 psig. or less are acceptable for storage in many containers. However, even lower vapor pressures of about 0.1 psig. or less are preferred, while pressures of about 1 atmosphere absolute or less at 20° C. are most preferred for storage in either open or closed containers. Thus, solutions having vapor pressures only slightly above 1 atmosphere absolute at 20° C. can be treated by the described methods with resulting improvements in storage stability. For that matter, solutions containing any amount of residual carbonate, even those having equilibrium vapor pressures of about 1 atmosphere or less at 20° C., can be beneficially treated by these methods since such treatment further reduces total carbonate content and reduces equilibrium vapor pressure at temperatures above 20° C. Thus, the stripped solutions will generally have an equilibrium vapor pressure of 0.5 psig. or less, preferably about 0.1 psig. or less, and most preferably about 1 atmosphere absolute or less at 20° C. In terms of total carbonate concentration, the most preferred equilibrium vapor pressures of about 1 atmosphere absolute or less correspond to total carbonate concentrations equal to or below the saturation limit of an equivalent amount of carbon dioxide in the solution at 20° C. and 1 atmosphere pressure absolute. In other words, the total carbonate concentration is most preferably below the level which would generate sufficient carbon dioxide to exceed the saturation limit in the solution at 20° C. and 1 atmosphere pressure absolute, since that results in the most preferred solutions having equilibrium vapor pressures of about 1 atmosphere absolute or less at 20° C. Since the solubility of the carbon dioxide in such solutions varies as a function of solution composition, e.g. concentration of urea and identity and concentration of acid and other components, the tolerable amount of total carbonate in the finished solutions will vary with composition. Generally, however, total carbonate content will be equivalent to about 0.2 weight percent or less, preferably about 0.1 weight percent or less expressed as equivalent carbon dioxide. Acceptably low total carbonate concentrations can be identified by storing samples of the treated solution in closed containers in which the solution occupies 90 percent of the container volume for a suitable period of time, e.g. 48 hours, 1 week or longer, as desired, at 20° C. (or higher temperature if higher temperatures are to be employed) and measuring the pressure existing above the solution. However, total carbonate concentrations which exceed that acceptable for long-term storage generally can be identified within 48 hours of storage.

While the problems associated with gradual carbon dioxide evolution exist in the more dilute solutions described, they are even more severe with more concentrated solutions. Accordingly, these methods are useful for the treatment of solutions in which the urea and acid, in combination, constitute at least about 5 weight percent of the solution, and they are even more beneficial when the urea and acid, in combination, constitute at least about 10, preferably at least about 20, and most preferably at least about 50 weight percent of the solution. Typically, the urea and acid, in combination, will constitute about 5 to about 80, most often about 10 to about 80 weight percent of the solution, particularly when sulfuric and/or phosphoric acids are employed.

The finished solutions can contain one or more of numerous materials which contribute to the quality of the solution, such as surfactants, major and minor nutrients, non-aqueous diluents and solvents other than water, such as alcohols, ethers, etc. Insoluble matter such as protein, cellulose and/or other polysaccharides or polyesters can also be present as described in U.S. Patent 4,722,986, referred to above, and U.S. Patent 4,664,717, METHODS FOR HYDROLYZING POLYSACCHARIDES AND COMPOSITIONS USEFUL THEREIN, issued May 12, 1987, the disclosure of which is incorporated herein in its entirety. Such materials can also be present in the solutions prior to stripping provided they do not interfere with the ability of the stripping procedure to reduce carbonate content to an acceptable level. Materials, such as surfactants, which cause solution foaming are preferably not present during stripping for obvious reasons.

The more dilute of the above-described acidic urea solutions can be prepared by simply adding acid to a urea solution, or vice versa by any acceptable means. However, manufacture of more concentrated solutions, e.g. those in which the urea and sulfuric acid, in combination, constitute at least about 30 weight percent of the solution and which have relatively high acid concentrations, are preferably manufactured by procedures which provide sufficient cooling to control the heat evolved by the exothermic reaction that occurs between urea and some acids, such as sulfuric acid, and/or the heat of acid dilution. For instance, concentrated urea-sulfuric acid solutions can be safely and efficiently prepared by the methods described in U.S. Patent 4,445,925, referred to above.

The invention is further described by the following examples which are illustrative of specific modes of practicing the invention and are not intended as limiting the scope of the invention as defined by the appended claims.

### Example 1

Approximately 20 tons of an aqueous solution of urea and sulfuric acid reaction product was produced by the method described in U.S. Patent 4,445,925, referred to above, and stored in a vented tank for 1 day. The product contained 49 weight percent equivalent sulfuric acid, 32 weight percent equivalent urea, and 19 weight percent water and had a pH below 2. This composition has a urea/H₂SO₄ molar ratio of 1.06, and therefore consists, primarily, of the monourea adduct of sulfuric acid, i.e. monocarbamide dihydrogen sulfate. Briefly, the manufacturing procedure involved simultaneously adding the required amounts of urea, sulfuric acid and water to a 6500 gallon reactor containing a mixture of previously reacted material and freshly added reacting material. A recycle stream was continuously withdrawn from the reactor and passed through a direct contact, packed bed air cooler in which the reacting liquid phase of urea, sulfuric acid, and water, in addition to previously reacted material, was passed downwardly in direct contact with upwardly rising air under conditions sufficient to maintain the temperature of all of the material in the reaction zone at less than 80° C. The cooler comprised a bed of 2-inch INTERLOX^{R} polypropylene saddles approximately 5.7 feet deep and 10 feet in diameter over which the urea-sulfuric acid composition was passed downwardly into contact with upwardly rising air.

Following approximately 1 day of storage, approximately 10 tons of the composition were returned to the reactor and recycled through the direct contact air cooler at a rate corresponding to 26 passes of the total reactor volume per hour with an air flow rate of approximately 20,000 cubic feet per minute. Recirculation through the cooler, which in this operation acted as a stripper, was continued for 3.5 hours.

The composition was sampled before and after stripping, and the samples were maintained in gas-tight pressure vessels. The vapor pressure of each sample was then monitored with a water manometer for 17 days, and these results are reported in Table 1.

**TABLE 1**

| Sample No. | Stripping Time, Min. | Vapor Pressure, cm. H₂O | | | | |
|---|---|---|---|---|---|---|
| | | Day 0 | Day 1 | Day 2 | Day 3 | Day 17 |
| 1 | 0 | 0 | Na^{a} | >100 | >100^{b} | >100^{b} |
| 2 | 20 | 0 | 0 | 0 | 0 | 0 |

| | | | | | | |
|---|---|---|---|---|---|---|
| ^{a} Observation inadvertently omitted | | | | | | |
| ^{b} Significant increases not observed after Day 2, possibly due to CO₂ adsorption into and venting through manometer. | | | | | | |

These results demonstrate that material that had been manufactured in the described reactor-cooler system and stored in a vented tank for 1 day developed a vapor pressure of greater than 100 centimeters water (greater than 1.5 psig.) in a closed container within 2 days. (The first day observation for the unstripped material was inadvertently omitted.) Thus, the manufacturing method, which also involves passage of the material through the packed bed cooler, for some reason, is not adequate to prevent vapor pressure buildup in acidic urea solutions. This inadequacy is due to the conditions maintained during the reaction which are necessary for production of the urea-sulfuric acid composition. In contrast, recirculation of the previously manufactured composition through the stripper for 20 minutes (9 cycles) produced a composition which generated no observable vapor pressure even after storage for 17 days.

### Example 2

The equilibrium vapor pressure of an aqueous solution containing 20 weight percent urea and 10 weight percent orthophosphoric acid having a pH below 2 can be reduced to a level below 0.5 psig. by agitating 10 gallons of the solution in a 30 gallon vessel with a 4-inch diameter impeller mixer at 300 rpm for 30 minutes while sparging air into the bottom of the vessel and below the liquid surface at a rate of 0.5 standard cubic foot per minute.

### Example 3

The equilibrium vapor pressure of an aqueous solution containing 20 weight percent urea and 10 weight percent acetic acid having a pH below 2 can be reduced to a level below 0.5 psig. by stripping the solution for 30 minutes by the procedure described in Example 2.

## Claims

1. A method for forming an acidic, low-vapor pressure, aqueous urea solution having a pH of about 5.5 or less and an equilibrium vapor pressure at 20°C of 0.5 psig or less which method comprises stripping an aqueous solution comprising at least about 5 weight percent urea, a total carbonate content which, if present as carbon dioxide, would produce an equilibrium vapor pressure over said solution of more than 0.5 psig at 20°C, and sufficient acid to produce a pH of about 5.5 or less under conditions sufficient to reduce said total carbonate content to a level which, if present as carbon dioxide, would produce an equilibrium vapor pressure over said solution of 0.5 psig or less at 20°C.

2. The method according to Claim 1, wherein said solution has a pH of about 2 or less, contains at least about 1 weight percent of said acid, and is stripped under conditions sufficient to reduce the total carbonate content thereof to a level which, if present as carbon dioxide, would produce an equilibrium vapor pressure over said solution of about 1 atmosphere absolute or less at 20°C.

3. The method according to Claim 1 or Claim 2, wherein said solution comprises at least about 10 weight percent urea.

4. The method according to Claim 1, wherein said acid comprises a member selected from the group consisting of sulfuric and phosphoric acids or a combination thereof.

5. A method for producing an aqueous urea solution having a pH of about 5.5 or less and an equilibrium vapor pressure of 1 atmosphere absolute or less at 20°C which method comprises stripping an aqueous solution containing at least about 5 weight percent urea, sufficient acid to produce a pH of about 5.5 or less, and a total carbonate content which, if present as carbon dioxide, would result in an equilibrium vapor pressure greater than 1 atmosphere absolute at 20°C, under conditions sufficient to produce said solution having an equilibrium vapor pressure of 1 atmosphere absolute or less at 20°C.

6. The method according to Claim 1 or Claim 5, wherein said solution has a pH of about 2 or less.

7. The method according to Claim 5, wherein said solution has a pH of about 2 or less and comprises at least about 10 weight percent urea and at least about 1 weight percent of said acid.

8. The method according to Claim 5, wherein said solution comprises at least about 10 weight percent urea and at least about 5 weight percent of said acid.

9. The method according to Claim 5, wherein said solution comprises at least about 20 weight percent urea and at least about 10 weight percent of said acid, and said acid comprises a member selected from the group consisting of sulfuric acid, phosphoric acid or a combination thereof.

10. The method according to Claim 1 or Claim 5, wherein said urea and said acid, in combination, constitute about 50 weight percent or more of said solution.

11. The method according to any one of the preceding claims, wherein said urea comprises at least about 20 weight percent of said solution, said urea and acid, in combination, constitute at least about 30 weight percent of said solution, and said solution has a pH of about 2 or less.

12. The method according to any one of the preceding claims, wherein said solution comprises a member selected from the group consisting of the monourea adduct or sulfuric acid, the diurea adduct of sulfuric acid, or combinations thereof.

13. A method for storing an aqueous, acidic urea solution having a pH of about 5.5 or less which method comprises stripping an aqueous solution comprising sufficient acid to obtain pH of about 5.5 or less, about 10 weight percent urea or more, and a total carbonate content which, if present as carbon dioxide, would produce an equilibrium vapor pressure over said solution greater than 0.5 psig at 20°C under conditions sufficient to obtain a total carbonate content which, if present as carbon dioxide, would result in an equilibrium vapor pressure over said solution of 0.5 psig or less at 20°C, and storing the resulting stripped solution in an unvented container.

14. The method according to Claim 13, wherein said urea and acid, in combination, constitute at least about 20 weight percent of said solution.

15. The method according to Claim 13 or Claim 14, which further comprises adding a surfactant to said stripped solution before storing in said unvented container.

16. The method according to Claim 13, wherein said urea and acid, in combination, constitute at least about 20 weight percent of said solution, said acid comprises a member selected from the group consisting of sulfuric acid, phosphoric acid or a combination thereof, and said method further comprises adding a surfactant to said stripped solution before storing the same.

17. A method according to any one of the preceding claims wherein the aqueous solution is stripped by a method selected from gas stripping, mechanical stripping or a combination thereof.

18. The method according to Claim 17, wherein said aqueous solution is stripped with a gas selected from the group consisting of air and nitrogen.

19. The method according to any one of the preceding claims, wherein the total carbonate content of the resulting stripped solution is about 0.1 weight percent or less.

20. An aqueous acidic urea solution comprising at least 20 weight percent urea, and a combined concentration of urea and acid corresponding to at least 30 weight percent of said solution and having an equilibrium vapor pressure over said solution of 0.5 psig or less at 20°C, and a pH of 5.5 or less.

21. The composition according to Claim 20, wherein said combined concentration of urea and acid corresponds to at least about 50 weight percent of said solution.

22. The composition according to any one of Claim 20 or Claim 21, wherein said acid is selected from the group consisting of sulfuric acid, phosphoric acid, and combinations thereof.

23. The composition according to Claim 20 or Claim 21, wherein said solution comprises a member selected from the group consisting of the monourea adduct of sulfuric acid, the diurea adduct of sulfuric acid, and combinations thereof.
